# EUROPEAN PATENT SPECIFICATION

(11) **EP 2 902 807 B1**
(45) Date of publication and mention of the grant of the patent: **01.05.2019**
(21) Application number: 13842340.5
(22) Date of filing: 10.09.2013
(51) Int. Cl.: G01T 1/202

(54) **RADIOGRAPH DETECTION DEVICE**
RÖNTGENSTRAHLDETEKTIONSVORRICHTUNG
DISPOSITIF DE DÉTECTION DE RADIOGRAPHE

(30) Priority: 27.09.2012 JP 2012213873; 29.07.2013 JP 2013156699
(43) Date of publication of application: 05.08.2015
(73) Proprietor: FUJIFILM Corporation, Tokyo 106-8620 (JP)
(72) Inventor: OKADA, Yoshihiro, Ashigarakami-gun Kanagawa 258-8538 (JP); NAKATSUGAWA, Haruyasu, Ashigarakami-gun Kanagawa 258-8538 (JP)
(74) Representative: Klunker IP Patentanwälte PartG mbB
(86) International application number: PCT/JP2013/074328
(87) International publication number: WO 2014/050533

(56) References cited:
- WO-A1-2012/090526
- JP-A- 2009 231 788
- JP-A- 2009 252 835
- JP-A- 2010 213 917
- JP-A- 2011 247 684
- JP-A- 2012 052 847
- JP-A- 2012 137 438
- US-A1- 2009 250 699

## Description

### TECHNICAL FIELD

The present invention relates to a radiographic image detection device according to the preamble of claim 1 for converting radiation into visible light. That is to say, it relates to an indirect-conversion type detection device.

### BACKGROUND ART

Recently, radiographic image detection devices have been used for diagnostic imaging in medical fields. The radiographic image detection device converts radiation (for example, X-rays) applied from a radiation source and passed through a region of interest of a subject (patient) into charges and produces a radiographic image. There are direct-conversion type and indirect-conversion type radiographic image detection devices. The direct-conversion type radiographic image detection device directly converts the radiation into the charges. The indirect-conversion type radiographic image detection device converts the radiation into visible light, and then converts the visible light into the charges.

The indirect-conversion type radiographic image detection device comprises a scintillator (phosphor layer) and a photoelectric conversion panel. The scintillator converts the radiation into the visible light. The photoelectric conversion panel detects the visible light and converts the detected visible light into the charges. The scintillator is made from cesium iodide (CsI) or gadolinium oxide sulfur (GOS). The photoelectric conversion panel is composed of an insulating substrate made from glass, and thin-film transistors and photodiodes arranged in a matrix over the surface of the insulating substrate.

Manufacturing cost using the CsI is more expensive than that using the GOS. However, the CsI has high efficiency of converting the radiation into the visible light. The CsI has a columnar crystal structure and due to its light-guide effect, the CsI is superior in SN ratio of image data. For these reasons, the CsI is particularly used for the scintillators of the high-end radiographic image detection devices.

"Laminated type" and "direct vapor deposition type" radiographic image detection devices, which utilize the CsI as the scintillator, are known. In the laminated type, a vapor deposition base, on which the scintillator is vapor-deposited, and the photoelectric conversion panel are adhered to each other through an adhesive layer such that the scintillator faces the photoelectric conversion panel. In the laminated type, end portions of the columnar crystals of the CsI are in close proximity to the photoelectric conversion panel. The visible light released from the end portions enters the photoelectric conversion panel efficiently, so that a radiographic image with high resolution is produced. However, the use of the vapor deposition base in the laminated type increases manufacturing processes and results in high cost.

In the direct vapor deposition type, the scintillator is directly vapor-deposited on the photoelectric conversion panel. The vapor deposition base is unnecessary in the direct vapor deposition type, so that the direct vapor deposition type has few manufacturing processes and low cost. Since the end portions of the columnar crystals of the CsI in the direct vapor deposition type are disposed opposite to the photoelectric conversion panel, the image quality of the radiographic image is inferior to that of the laminated type, but superior to that of the case where the scintillator is made from the GOS. Thus the direct vapor deposition type offers an excellent balance between performance and cost.

The direct vapor deposition type radiographic image detection devices of ISS (Irradiation Side Sampling) type are known. In the ISS type, of the photoelectric conversion panel and the scintillator accommodated in the housing, the photoelectric conversion panel is disposed on the radiation source side to allow the radiation from the radiation source to enter the scintillator through the photoelectric conversion panel (see Japanese Patent Laid-Open Publication No. 2012-105879). In the ISS type, the scintillator generates the light in portions on the photoelectric conversion panel side, so that radiographic images excellent in image quality and brightness are produced. In the ISS type, the thickness of the insulating substrate is reduced to improve the radiation transmitting property of the photoelectric conversion panel.

US 2009/250699 A1 discloses a radiographic image detection device. In this device, the scintillator and the photoelectric conversion panel are disposed in this order from a radiation incidence side. In the second planarizing film there are formed contact holes which are connected to the upper electrode of the photodiodes.

WO 2012/090526 A discloses a radiographic image detection device in which the photoelectric conversion panel and the scintillator are disposed in this order from the radiation incidence side. Further, on a vapor deposition area, cesium iodide is vapor-deposited.

### SUMMARY OF THE INVENTION

### Problems to be Solved by the Invention

However, the ISS type radiographic image detection device of the direct vapor deposition type has a problem that the scintillator easily comes off from the photoelectric conversion panel. There may be first to third reasons for this as follows.

The first reason is that the thermal expansion coefficient of the photoelectric conversion panel significantly differs from that of the scintillator (CsI) in the order of one digit. The second reason is that the photoelectric conversion panel, being placed in the proximity of the housing in the ISS type, is likely bend due to the load on the housing. The third reason is that the thin photoelectric conversion panel in the ISS type bends easily. Since the photoelectric conversion panel bends significantly at its edge portions, the scintillator is likely to come off particularly from the edge portions.

An object of the present invention is to provide an ISS type radiographic image detection device which prevents a scintillator from coming off from a photoelectric conversion panel easily.

### Means for Solving the Problems

In order to achieve the above and other objects, the radiographic image detection device of the present invention comprises the features of claim 1.

### MODE FOR CARRYING OUT THE INVENTION

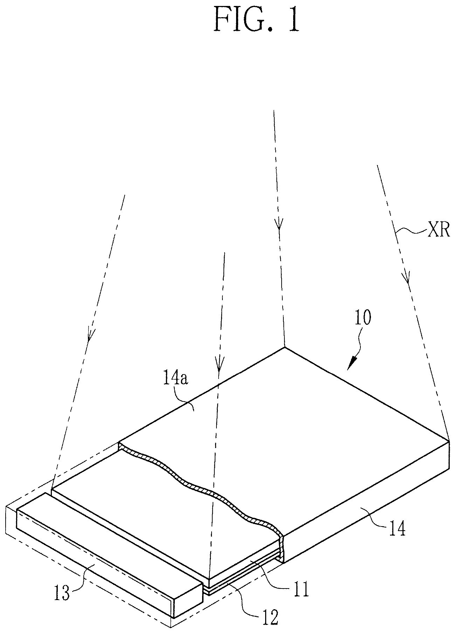

In Fig. 1, an X-ray image detection device 10 comprises a flat panel detector (FPD) 11, a support plate 12, a control unit 13, and a housing 14. The housing 14 accommodates the FPD 11, the support plate 12, and the control unit 13. The housing 14 has an integral monocoque structure made from lightweight carbon fiber reinforced plastics (carbon fiber) having high X-ray (XR) transmission property and high durability.

One of the sides of the housing 14 is formed with an opening (not shown). The FPD 11, the circuit plate 12, and the control unit 13 are inserted into the housing 14 through the opening at the time of manufacture of the X-ray image detection device 10. A lid (not shown) is attached so as to cover the opening after the insertion.

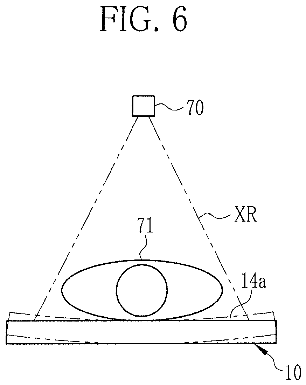

A top surface 14a of the housing 14 is an exposure surface on which X-rays XR emitted from an X-ray source 70 (see Fig. 6) and passed through a subject (patient) 71 (see Fig. 6) are applied at the time of imaging. The exposure surface 14a is provided with alignment mark(s) (not shown) to align the X-ray source 70 and the subject 71.

The size of the X-ray image detection device 10 is substantially the same as that of a conventional X-ray film cassette, and the X-ray image detection device 10 can be used in place of the conventional X-ray film cassette. For these reasons, the X-ray image detection device 10 is referred to as "electronic cassette".

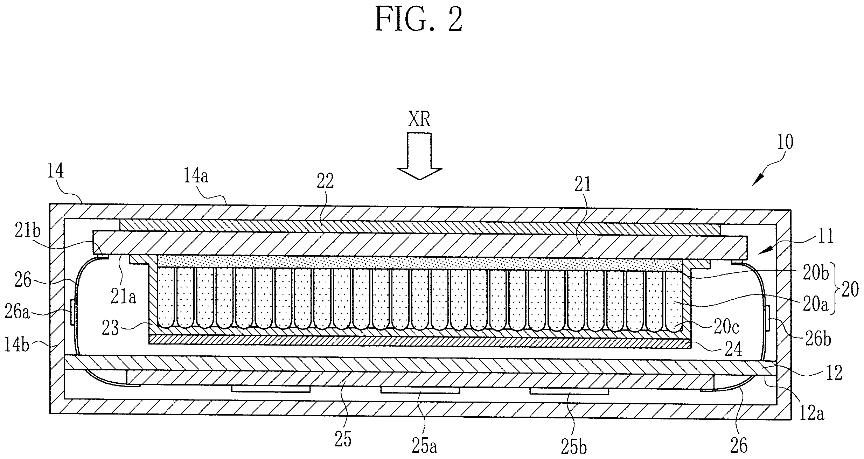

The FPD 11 and the support plate 12 are disposed in the housing 14 in this order from the exposure surface 14a side, to which the X-rays XR are applied at the time of imaging. The support plate 12 supports a circuit board 25 (see Fig. 2) and is fixed to the housing 14 with screws or the like. The control unit 13 is disposed near one of the shorter sides of the housing 14.

The control unit 13 accommodates a microcomputer and a battery (both not shown). The microcomputer communicates with a console (not shown), which is connected to the X-ray source 70, through a wired or wireless communicator (not shown), thereby controlling the operation of the FPD 11.

In Fig. 2, the FPD 11 comprises a scintillator 20 and a photoelectric conversion panel 21. The scintillator 20 converts the X-rays XR into visible light. The photoelectric conversion panel 21 converts the visible light into charges. The X-ray image detection device 10 is of ISS (Irradiation Side Sampling) type. The photoelectric conversion panel 21 and the scintillator 20 are disposed in this order from the side (the exposure surface 14a side) on which the X-rays XR are incident at the time of imaging. The scintillator 20 converts the X-rays XR which have passed through the photoelectric conversion panel 21 into the visible light, and releases the visible light. The photoelectric conversion panel 21 photoelectrically converts the visible light released from the scintillator 20 into the charges.

The photoelectric conversion panel 21 is affixed to the exposure surface 14a side of the housing 14 through an adhesive layer 22. The adhesive layer 22 is made from epoxy resin or the like.

The scintillator 20 is formed by vapor deposition of thallium-activated cesium iodide (CsI:Tl) on a surface 21a of the photoelectric conversion panel 21. The scintillator 20 has a plurality of columnar crystals 20a and a non-columnar crystal layer 20b. The non-columnar crystal layer 20b is formed on the photoelectric conversion panel 21 side. The columnar crystals 20a grow from the non-columnar crystal layer 20b. The columnar crystals 20a have their end portions 20c on the opposite side of the non-columnar crystal layer 20b.

The plurality of columnar crystals 20a are formed on the non-columnar crystal layer 20b. Each columnar crystal 20a is spaced from its adjacent columnar crystal 20a by an air layer. The refractive index of the columnar crystal 20a is approximately 1.81, which is greater than the refractive index (approximately 1.0) of the air layer, so that the columnar crystal 20a has light-guide effect. Due to the light-guide effect, most of the visible light generated in each columnar crystal 20a is transmitted in the columnar crystal 20a where the visible light is generated and enters the photoelectric conversion panel 21 through the non-columnar crystal layer 20b.

A sealing film 23 is formed over the scintillator 20. The sealing film 23 seals the columnar crystals 20a and the non-columnar crystal layer 20b. The sealing film 23 is formed from polyparaxylene, which is resistant to moisture, for example, Parylene C (the name of a product manufactured by Japan Parylene Co. ; "Parylene" is a registered trademark) . The sealing film 23 makes the scintillator 20 moisture-proof.

A light-reflecting film 24 is formed over the surface of the sealing film 23 that covers the end portions 20c of the columnar crystals 20a. The light-reflecting film 24 is formed of an aluminum film or vapor-deposited aluminum film. The light-reflecting film 24 reflects the visible light, released from the end portions 20c of the columnar crystals 20a, back into to the columnar crystals 20a. Thereby, efficiency for converting the X-rays XR into charges is improved.

The support plate 12 is disposed on the opposite side of the X-ray incidence side of the scintillator 20. The support plate 12 faces the light-reflecting film 24 through the air layer. The support plate 12 is fixed to sides 14b of the housing 14 with screws or the like. The circuit board 25 is fixed, using an adhesive or the like, to an underside 12a, which is located on the opposite side of the scintillator 20, of the support plate 12.

The circuit board 25 and the photoelectric conversion panel 21 are electrically connected to each other through a flexible printed circuit board 26. The flexible printed circuit board 26 is connected to an external terminal 21b, which is provided at an end of the photoelectric conversion panel 21, by a so-called TAB (Tape Automated Bonding) method.

A gate driver 26a and a charge amplifier 26b are mounted as integrated circuit (IC) chips on the flexible printed circuit board 26. The gate driver 26a drives the photoelectric conversion panel 21. The charge amplifier 26b converts the charge, which is outputted from the photoelectric conversion panel 21, into a voltage signal. A signal processor 25a and an image memory 25b are mounted on the circuit board 25. The signal processor 25a generates image data based on the voltage signals converted by the charge amplifier 26b. The image memory 25b stores the image data.

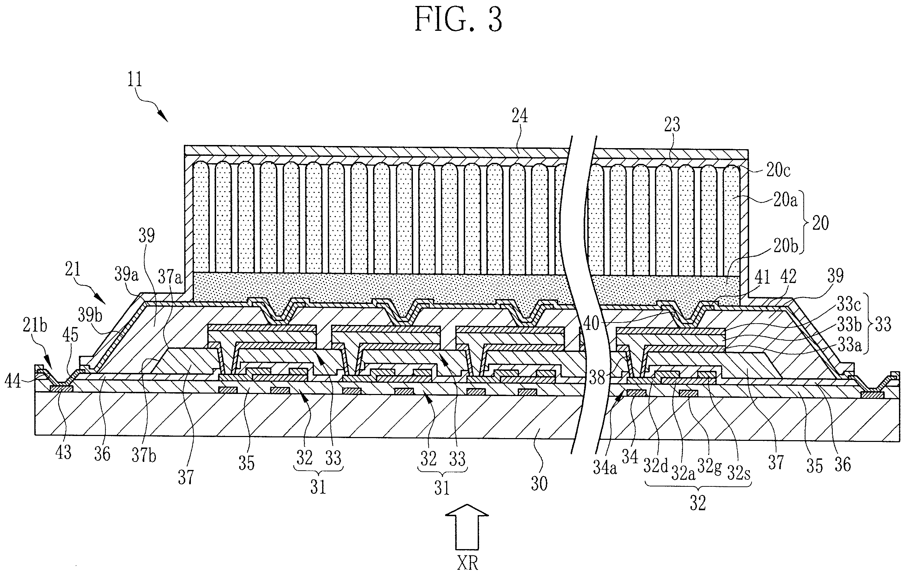

In Fig. 3, the photoelectric conversion panel 21 comprises an insulating substrate 30 made from non-alkali glass or the like and a plurality of pixels 31 arranged over the insulating substrate 30. It is preferred that the thickness of the insulating substrate 30 is less than or equal to 0.5 mm to improve X-ray (XR) transmission property.

Each pixel 31 has a thin film transistor (TFT) 32 and a photodiode (PD) 33 connected to the TFT 32. The PD 33 photoelectrically converts the visible light, which is generated by the scintillator 20, into a charge and stores the charge. The TFT 32 is a switching element, which reads out the charge stored in the PD 33.

The TFT 32 comprises a gate electrode 32g, a source electrode 32s, a drain electrode 32d, and an active layer 32a. The TFT 32 is of an inverted staggered type, in which the gate electrode 32g is disposed in a layer located below the source electrode 32s and the drain electrode 32d. The gate electrode 32g is formed over the insulating substrate 30. A charge storage electrode 34 is formed over the insulating substrate 30 to increase a charge storage capacity of each pixel 31. A ground voltage is applied to the charge storage electrode 34.

An insulating film 35, which is made from silicon nitride (SiNx) or the like, is formed over the insulating substrate 30 so as to cover the gate electrode 32g and the charge storage electrode 34. The active layer 32a is disposed over the insulating film 35 so as to face the gate electrode 32g. The source electrode 32s and the drain electrode 32d are disposed apart from each other by a predetermined distance over the active layer 32a. A part of the drain electrode 32d extends over the insulating film 35. The drain electrode 32d is opposed to the charge storage electrode 34 through the insulating film 35, thereby constituting a capacitor 34a.

The gate electrode 32g, the source electrode 32s, the drain electrode 32d, and the charge storage electrode 34 are formed from aluminum (Al) or cupper (Cu). The active layer 32a is formed from amorphous silicon. A TFT protecting film 36, which is made from silicon nitride (SiN_{X}) or the like, is formed over the insulating film 35 so as to cover the source electrode 32s, the drain electrode 32d, and the active layer 32a.

A first planarizing film 37, which has a flat surface, is formed over the TFT protecting film 36 so as to planarize the unevenness caused by the TFT 32. The first planarizing film 37 is formed with the thickness of 1 to 4 µm by applying photosensitive organic material of low permittivity (dielectric constant εᵣ = 2 to 4) (for example, positive-type photosensitive acrylic-based resin: material in which naphthoquinone-diazide-based positive-type photosensitive agent is mixed in base polymer composed of copolymer of methacrylic acid and glycidyl methacrylate, or the like).

A contact hole 38, which is in a position facing the drain electrode 32d, is formed through the first planarizing film 37 and the TFT protecting film 36. The PD 33 is connected to the drain electrode 32d of the TFT 32 through the contact hole 38. The PD 33 is composed of a lower electrode 33a, a semiconductor layer 33b, and an upper electrode 33c.

The lower electrode 33a is formed over the first planarizing film 37 so as to cover the inside of the contact hole 38 and also to cover the TFT 32. The lower electrode 33a is connected to the drain electrode 32d. The lower electrode 33a is formed from aluminum (Al) or indium tin oxide (ITO). The semiconductor layer 33b is stacked over the lower electrode 33a. The semiconductor layer 33b is made from PIN-type amorphous silicon, and comprises n⁺ layer, i layer, and p⁺ layer stacked from the bottom. The upper electrode 33c is formed over the semiconductor layer 33b. The upper electrode 33c is formed from material with high light-transmitting property such as indium tin oxide (ITO) or indium zinc oxide (IZO).

A second planarizing film 39 with a flat surface is formed over the PD 33 and the first planarizing film 37 so as to planarize the unevenness caused by the PD 33. The second planarizing film 39 is formed by applying photosensitive organic material, which is similar to the material of the first planarizing film 37, with the thickness of 1 to 4 pm.

A contact hole 40 is formed through the second planarizing film 39 so as to expose the upper electrode 33c. A bias line 41 is connected to the upper electrode 33c through the contact hole 40. The bias line 41 is connected to and shared by the upper electrode 33c of each of the PDs 33, and applies a bias voltage to the upper electrodes 33c. The upper electrode 33c is formed from aluminum (Al) or cupper (Cu).

An insulating protective film 42 is formed over the second planarizing film 39 and the bias line 41. The insulating protective film 42 is formed from silicon nitride (SiN_{X}) or the like, as in the case of the TFT protecting film 36.

As described above, the first planarizing film 37 is formed by applying the organic material. For this reason, an edge face 37b located outside of an edge (first edge) 37a of the first planarizing film 37 is inclined and has a tapered shape. In like manner, an edge face 39b located outside of an edge (second edge) 39a of the second planarizing film 39 is inclined and has a tapered shape. The first edge 37a is an outermost portion (a boundary with the edge face 37b) of the flat portion of the first planarizing film 37. The second edge 39a is an outermost portion (a boundary with the edge face 39b) of the flat portion of the second planarizing film 39. The second edge 39a is located outside of the first edge 37a. The above-mentioned external terminal 21b is provided outside of the second edge 39a.

The external terminal 21b is composed of a terminal electrode 43 and a metal film 45. The terminal electrode 43 is formed over the insulating substrate 30. The metal film 45 is provided to cover a contact hole 44 formed through the insulating film 35 and the TFT protecting film 36.

The bias line 41 is connected to the external terminal 21b for supplying the bias voltage, through wiring (not shown) disposed over the edge face 39b. The wiring is vapor-deposited over the edge face 39b. In a case where the edge face 39b has a steep inclination, the film thickness is reduced and the wiring may be broken. To prevent this, it is preferred that the edge face 39b has a gentle slope.

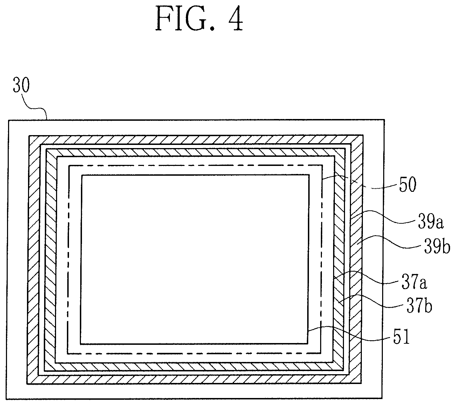

The scintillator 20 is formed over the flat surface of the second planarizing film 39 through the insulating protective film 42. To be more specific, as illustrated in Fig. 4, a vapor deposition area 50 of the scintillator 20 is located inside the second edge 39a and the first edge 37a and covers a pixel 31-formed area 51 in which the pixels 31 are formed.

The non-columnar crystal layer 20b is formed through vacuum vapor deposition over the insulating protective film 42 in the vapor deposition area. The non-columnar crystal layer 20b is composed of a plurality of particulate crystals and has small gaps between them (the non-columnar crystal layer 20b has a high space-filling ratio). For this reason, the non-columnar crystal layer 20b is highly adhesive to the insulating protective film 42. The thickness of the non-columnar crystal layer 20b is in the order of 5 µm. The columnar crystals 20a grow on the non-columnar crystal layer 20b through the vacuum vapor deposition. The diameter of the columnar crystal 20a is in the order of 6 µm, and substantially constant in the lengthwise direction of the columnar crystal 20a.

As described above, the sealing film 23 is formed to surround the scintillator 20. The sealing film 23 also covers an area outside the edge face 39b. The light-reflecting film 24 is formed over the sealing film 23 as described above.

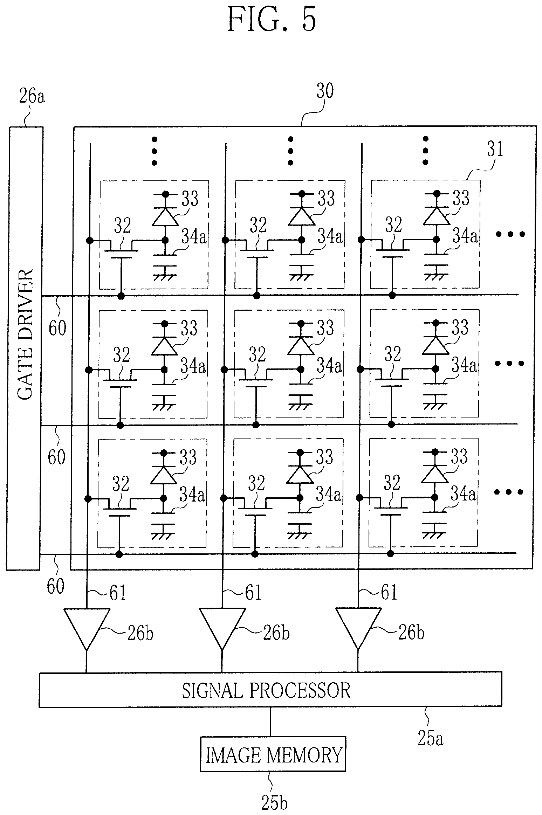

In Fig. 5, the pixels 31 are arranged in a two-dimensional matrix over the insulating substrate 30. As described above, each pixel 31 comprises the TFT 32, the PD 33, and the capacitor 34a. Each pixel 31 is connected to a gate line 60 and a data line 61. The gate line 60 extends in a row direction. The plurality of gate lines 60 are arranged in a column direction. The data line 61 extends in the column direction. The plurality of data lines 61 are arranged in the row direction to cross the gate lines 60. The gate line 60 is connected to the gate electrode 32g of the TFT 32. The data line 61 is connected to the drain electrode 32d of the TFT 32.

An end of the gate line 60 is connected to the gate driver 26a. An end of the data line 61 is connected to the charge amplifier 26b. The gate driver 26a sequentially provides the gate drive signal to each of the gate lines 60 to turn on the TFTs 32 connected to each gate line 60. In response to turning on the TFT 32, the charges stored in the PD 33 and the capacitor 34a are outputted to the data line 61.

The charge amplifier 26b has a capacitor (not shown) for storing the charge. The charge amplifier 26b integrates the charge outputted to the data line 61, and converts the charge into a voltage signal. The signal processor 25a performs A/D conversion, gain correction processing, and the like on the voltage signal outputted from the charge amplifier 26b, to generate image data. The image memory 25b is composed of a flash memory or the like, and stores the image data generated by the signal processor 25a. The image data stored in the image memory 25b is readable externally through a wired or wireless communicator (not shown).

Next, an operation of the X-ray image detection device 10 is described. To perform imaging using the X-ray image detection device 10, an operator (e.g. radiologic technologist) places the subject 71 on the X-ray image detection device 10 and places the X-ray source 70 so as to face the subject as illustrated in Fig. 6.

The console is operated to command the start of imaging. In response to this, the X-ray source 70 emits the X-rays XR. The X-rays XR passed through the subject 71 are applied to the exposure surface 14a of the X-ray image detection device 10. The X-rays XR applied to the exposure surface 14a pass through the housing 14, the adhesive layer 22, and the photoelectric conversion panel 21 in this order, and then enters the scintillator 20.

The scintillator 20 absorbs the X-rays XR and generates the visible light. In the scintillator 20, the visible light is mostly generated inside the columnar crystal 20a on the non-columnar crystal layer 20b side. The visible light generated in the columnar crystals 20a is transmitted through the respective columnar crystals 20a due to the light-guide effect, and then passes through the non-columnar crystal layer 20b. Thereafter, the visible light enters the photoelectric conversion panel 21. The visible light which has been transmitted inside the columnar crystal 20a to the end portion 20c and emitted from the end portion 20c is reflected back into the columnar crystal 20a by the light-reflecting film 24 and then passes through the non-columnar crystal layer 20b and enters the photoelectric conversion panel 21.

The PD 33 of each pixel 31 converts the visible light, which has entered the photoelectric conversion panel 21, into a charge. The charge is stored in the PD 33 and the capacitor 34a. In response to the completion of the X-ray emission from the X-ray source 70, the gate driver 26a applies the gate drive signals sequentially to the gate electrodes 32g of the TFTs 32 through the gate lines 60. Thereby the TFTs 32, arranged in the row direction, are turned on sequentially in the column direction. The charges stored in the PDs 33 and the capacitors 34a are outputted to the data line 61 through the turned-on TFTs 32.

The charge amplifier 26b converts the charges, which have been outputted to the data line 61, into voltage signals and inputs the voltage signals to the signal processor 25a. The signal processor 25a generates the image data based on the voltage signals of all the pixels 31. The image data is stored in the image memory 25b.

During the imaging, the X-ray image detection device 10 may bend slightly due to the weight of the subject 71 as illustrated by two-dot chain lines in Fig. 6. Since the X-ray image detection device 10 is of the ISS type, the photoelectric conversion panel 21 is disposed on the exposure surface 14a side, so that the weight of the subject 71 exerts on the photoelectric conversion panel 21 through the housing 14. The insulating substrate 30 of the photoelectric conversion panel 21 bends easily since the thickness of the insulating substrate 30 is small (less than or equal to 0.5 mm) so as to improve the X-ray (XR) transmission property. Furthermore, the housing 14 has a monocoque structure, which is excellent in lightweight but has low load-carrying capacity, so that the housing 14 is likely to bend due to the subject 71.

In this embodiment, the scintillator 20 does not come off easily from the photoelectric conversion panel 21 because the scintillator 20 is formed over the planarized surface of the second planarizing film 39 and located inside the first and second edges 37a and 39a of the first and second planarizing films 37 and 39. The scintillator 20 has the high space-filling ratio, and the non-columnar crystal layer 20b, which is highly adhesive to the photoelectric conversion panel 21, is directly vapor-deposited on the photoelectric conversion panel 21. Thereby the peeling of the scintillator 20 from the photoelectric conversion panel 21 is furthermore prevented. The pixel 31-formed area 51 over the photoelectric conversion panel 21 has minute projections and depressions caused by the bias line 41 and the like. However, the scintillator 20 is vapor-deposited also to a perfectly flat portion outside the pixel 31-formed area 51 so as to cover the pixel 31-formed area 51. Thereby, the peeling of the scintillator 20 from the photoelectric conversion panel 21 is prevented.

In an embodiment, the second edge 39a is disposed outside the first edge 37a. In a case where the second edge 39a is located inside the first edge 37a, residues of the second planarizing film 39 make the surface of the taper-shaped edge face 39b uneven, resulting in peeling and cracks in the insulating protective film 42. In this embodiment, this problems is prevented because the second edge 39a is disposed outside the first edge 37a.

In this embodiment, the scintillator 20 is not vapor-deposited over the edge faces 37b and 39b, which have uneven surfaces. Therefore the probability of the scintillator containing the abnormally-grown protrusions, which occur due to the abnormal growth of the columnar crystals on an uneven surface, is low.

The edge face 39b is formed by a coating method, so that the shape of its surface is actually unstable. The sealing film 23 is likely to come off easily in the case where the edge portion of the sealing film 23 is located on the edge face 39b. However, the sealing film 23 fully covers the edge face 39b of the second planarizing film 39 while sealing the scintillator 20. Thereby, the peeling is prevented.

The layer 32a of the TFT 32 is formed from the amorphous silicon. Alternatively, the active layer 32a may be formed from amorphous oxide (for example, In-O type), organic semiconductor material, carbon nanotube, or the like.

The semiconductor layer 33b of the PD 33 is formed from the amorphous silicon. Instead, the semiconductor layer 33b may be formed from organic photoelectric conversion material (e.g. quinacridone-based organic compound or phthalocyanine-based organic compound). The amorphous silicon has a wide absorption spectrum. The organic photoelectric conversion material has a sharp absorption spectrum in the visible range, so that it hardly absorbs electromagnetic waves other than the visible light generated in the scintillator 20. As a result, noise is reduced.

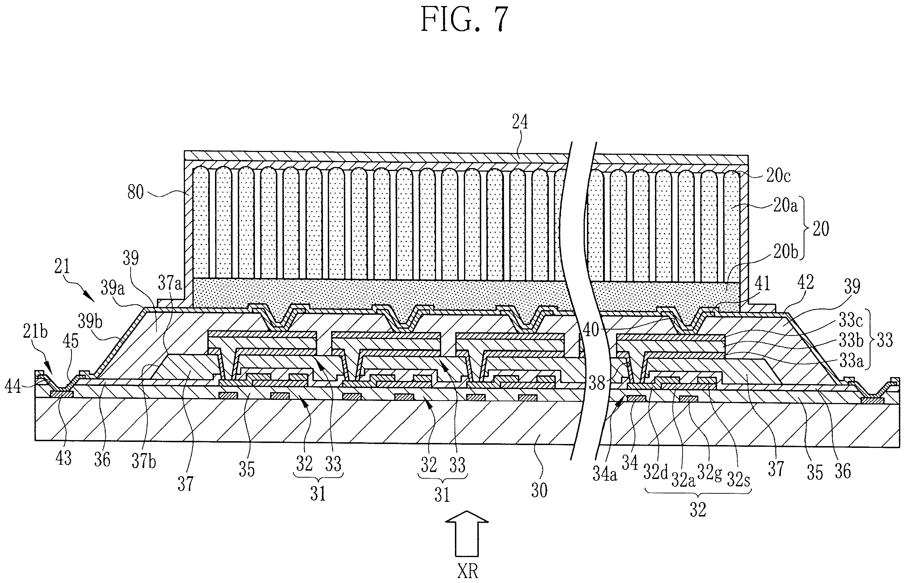

The sealing film 23 made from polyparaxylene is used. Instead, a sealing film may be made from PET (Polyethylene terephthalate) or an aluminum (Al) film. In this case, it is preferred that a sealing film 80 covers the scintillator 20, and an edge portion of the sealing film 80 is located inside the edge 39a of the second planarizing film 39 as illustrated in Fig. 7. The sealing film 80 may be formed by a vapor-deposition method using a mask or a hot-melt method.

AS illustrated in Fig. 3, the bias line 41, which is used for applying the bias voltage to the upper electrode 33c of the PD 33, is disposed above the PD 33 (in other words, on the scintillator 20 side). This configuration is referred to as the upper layer bias line structure. In the upper layer bias line structure, the scintillator 20 is formed by the vapor deposition over the bias line 41 through the insulating protective film 42. Since the scintillator 20 is formed from CsI:Tl, which is likely to become deliquescent by absorbing moisture, the deliquescence of the scintillator 20 may cause corrosion and deterioration of the bias line 41 through the insulating protective film 42. The corrosion and the deterioration of the bias line 41 may cause failure in the application of the bias voltage. To prevent the corrosion and the deterioration of the bias line 41, the thickness of the insulating protective film 42 may be increased. However, increasing the thickness of the insulating protective film 42 increases the distance between the PD 33 and the scintillator 20, resulting in image deterioration.

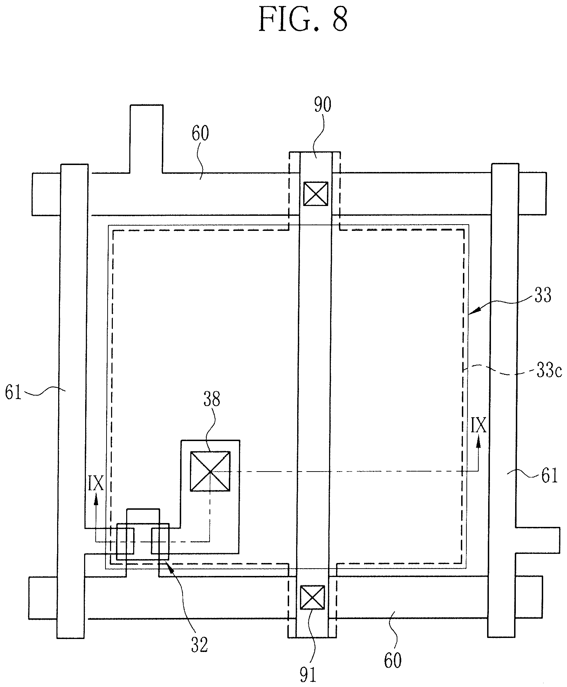

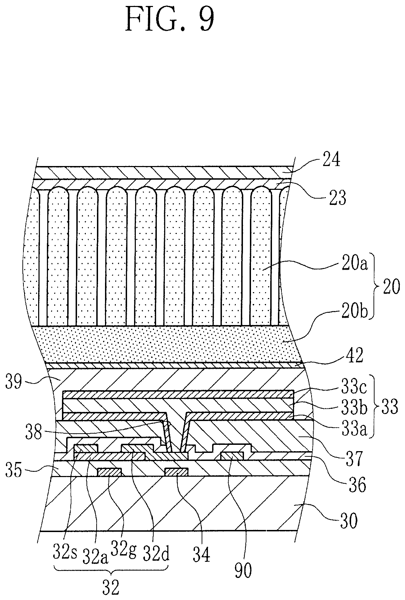

As illustrated in Figs. 8 and 9, a lower layer bias line structure, in which a bias line 90 is provided below the PD 33 (on the opposite side of the scintillator 20), is formed. The bias line 90, which is made from aluminum (AL) or cupper (Cu), is formed in the layer (between the insulating film 35 and the TFT protection film 36) in which the source electrode 32s and the drain electrode 32d of the TFT 32 are formed. The bias line 90 extends in a direction (column direction) along the data line 61. At the position of intersection with the gate line 60, the bias line 90 is connected, using a contact plug 91, to the upper electrode 33c of the PD 33.

Since the bias line 90 is disposed below the PD 33 in the lower layer bias line structure, the failure in the application of the bias voltage is prevented because the bias line 90 is not affected by the deliquescence of the scintillator 20. In the upper layer bias line structure, the bias line 41 is formed between the PD 33 and the scintillator 20, blocking a part of the visible light generated by the scintillator 20. Thereby, the light-receiving efficiency of the PD 33 is reduced. In the lower layer bias line structure, the bias line 90 is not disposed between the PD 33 and the scintillator 20. As a result, the light-receiving efficiency of the PD 33 is improved.

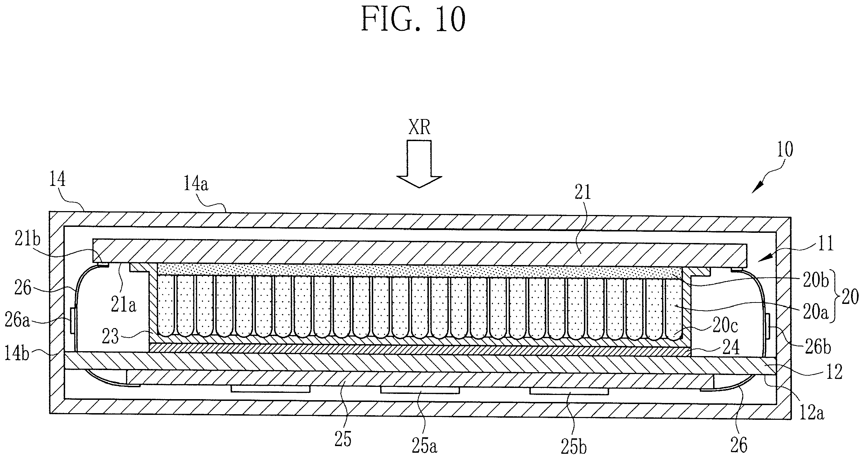

As illustrated in Fig. 2, the FPD 11 is affixed to the exposure surface 14a side of the housing 14 through the adhesive layer 22. As illustrated in Fig. 10, the FPD 11 may be fixed to the support plate 12. In this case, the adhesive layer 22 may be omitted. It is preferred that the support plate 12 is a carbon plate with the thickness in the order of 1 mm. The scintillator 20 side of the FPD 11 is affixed to the support plate 12 with an acrylic-based adhesive or the like. In this case, the adhesive may be applied to the entire surface or only to peripheral portions on the light-reflecting film 24 side of the scintillator 20.

The support plate 12 may have a laminated structure of carbon plate and buffer layer. It is preferred that the buffer layer is formed from polymeric material having adhesion property (e.g. isotactic polypropylene, poly-α-methylstyrene) or the like and faces the scintillator 20. In this case, the buffer layer protects the end portions 20c of the columnar crystals 20a from impact and the like.

In the case where the FPD 11 is fixed to the support plate 12 as described above, a module in which the FPD 11 is adhered to the support plate 12 may be produced in advance. The X-ray image detection device 10 may be produced by mounting the module composed of the FPD 11 and the support plate 12 inside the housing 14 .

In the above embodiment, the scintillator 20 is formed over the insulating protective film 42. A planarizing film is formed over the insulating protective film 42, and the scintillator 20 is formed over the planarizing film. The material of the planarizing film is preferred to be the material for forming a barrier described in Japanese translation of PCT application No. 2002-524841. For example, acrylic resin, polyimide resin, benzo-cyclo-butene (BCB)-based resin, silicone resin, polyparaxylene and its halogen derivatives such as polytetrafluoroparaxylene, tropicalizing varnish, sol-gel of a mineral compound, soluble silicates (known as "liquid glasses"), and polyester membrane are preferred.

In the above embodiment, the X-rays are used as the radiation. The radiation other than the X-rays, for example, gamma rays, alpha rays, or the like may be used. In the above embodiment, the present invention is described using an electronic cassette, being a portable radiographic image detection device, by way of example. The present invention is also applicable to a radiographic image detection device of standing type or lying type, mammography device, and the like.

## Claims

1. A radiographic image detection device (10) comprising a photoelectric conversion panel (21) and a scintillator (20), the photoelectric conversion panel and the scintillator being disposed in this order from a radiation incidence side on which radiation from a radiation source (70) is incident at the time of imaging,
(A) the photoelectric conversion panel comprising:
an insulating substrate (30);
a plurality of switching elements (32) formed over the insulating substrate;
a first planarizing film (37) formed to cover the switching elements and having a planarized surface;
a plurality of photodiodes (33) formed over the first planarizing film; and
a second planarizing film (39) formed to cover the photodiodes and the first planarizing film and having a planarized surface;
(B) the scintillator comprising:
a non-columnar crystal layer (20b) and a plurality of columnar crystals (20a) formed on the non-columnar crystal layer,
the non-columnar crystal layer being disposed on a photoelectric conversion panel side compared with the columnar crystals;
cesium iodide vapor-deposited on a vapor deposition area (50), the vapor deposition area extending over the second planarizing film and located inside a first edge (37a) of the first planarizing film and a second edge (39a) of the second planarizing film and covering an area (51) in which the switching elements and the photodiodes are formed; and
(C) a support plate (12) for supporting the scintillator, the support plate being fixed to a surface, of the scintillator, on which the photoelectric conversion panel is not disposed,
**characterized in that**
the photoelectric conversion panel has a bias line (90) for supplying a bias voltage to the each photodiode, and the bias line is formed between the each photodiode and the insulating substrate..

2. The radiographic image detection device according to claim 1, wherein the first edge of the first planarizing film is located inside the second edge of the second planarizing film.

3. The radiographic image detection device according to claim 1 or 2, wherein a plurality of pixels (31) each containing the one switching element and the one photodiode are arranged in a matrix over the insulating substrate.

4. The radiographic image detection device according to claim 3, wherein the switching element is an inverted staggered type TFT (32).

5. The radiographic image detection device according to claim 3 or 4, comprising a first protection film (36) between the switching element and the first planarizing film.

6. The radiographic image detection device according to claim 5, comprising a second protection film (42) between the second planarizing film and the scintillator.

7. The radiographic image detection device according to any one of claims 1 to 6, wherein an edge face (37b) of the first planarizing film and an edge face (39b) of the second planarizing film are taper-shaped.

8. The radiographic image detection device according to any one of claims 1 to 7, comprising a sealing film (23) for covering the surface of the scintillator and the edge face of the second planarizing film.

9. The radiographic image detection device according to claim 8, comprising a light-reflecting film (24) over the sealing film.

10. The radiographic image detection device according to any one of claims 1 to 9, wherein the photoelectric conversion panel, the scintillator, and the support plate are accommodated in a housing (14) with a monocoque structure.

11. The radiographic image detection device according to any one of claims 1 to 10, wherein the insulating substrate is made from glass.

## Patentansprüche

1. Röntgenbild-Detektorvorrichtung (10), umfassend eine photoelektrische Umwandlungsplatte (21) und einen Scintillator (20), die in dieser Reihenfolge von einer Strahlungseinfallseite, auf der Strahlung von einer Strahlungsquelle (70) zur Zeit der Bildgebung einfällt, angeordnet sind, wobei
(A) die photoelektrische Wandlerplatte umfasst:
ein isolierendes Substrat (30);
mehrere Schaltelemente (32), die über dem isolierenden Substrat gebildet sind;
eine erste Planarisierungsschicht (37), ausgebildet zum Bedecken der Schaltelemente und mit einer planarisierten Oberfläche ausgestattet;
mehrere Photodioden (33), die über der ersten Planarisierungsschicht ausgebildet sind; und
eine zweite Planarisierungsschicht, ausgebildet zum Abdecken der Photodioden und der ersten Planarisierungsschicht, und mit einer planarisierten Oberfläche ausgestattet;
(B) der Scintillator umfasst:
eine nicht-säulenförmige Kristallschicht (20b) und mehrere säulenförmige Kristalle (20a) auf der nicht-säulenförmigen Kristallschicht,
wobei die nicht-säulenförmige Kristallschicht auf der Seite der photoelektrischen Wandlerplatte im Hinblick auf die säulenförmigen Kristalle angeordnet ist;
Cäsium-lodid, aufgedampft auf einen Aufdampfungsbereich (50), der sich über die zweite Planarisierungsschicht erstreckt und sich im Inneren einer ersten Kante (37a) der ersten Planarisierungsschicht und einer zweiten Kante (39a) der zweiten Planarisierungsschicht befindet, und der einen Bereich (51) abdeckt, in welchem die Schaltelemente und die Photodioden ausgebildet sind; und
(C) eine Trägerplatte (12) zum Lagern des Scintillators vorgesehen ist, die an einer Oberfläche des Scintillators fixiert ist, auf der sich nicht die photoelektrische Wandlerplatte befindet,
**dadurch gekennzeichnet, dass**
die photoelektrische Wandlerplatte eine Vorspannleitung (90) aufweist, um jeder Photodiode eine Vorspannung zuzuführen, und die Vorspannleitung zwischen jeder Photodiode und dem isolierenden Substrat ausgebildet ist.

2. Vorrichtung nach Anspruch 1, bei der die erste Kante der ersten Planarisierungsschicht sich im Inneren der zweiten Kante der zweiten Planarisierungsschicht befindet.

3. Vorrichtung nach Anspruch 1 oder 2, bei der die mehreren Pixel (31), die jeweils das eine Schaltelement und die eine Photodiode enthalten, in einer Matrix über dem isolierenden Substrat angeordnet sind.

4. Vorrichtung nach Anspruch 3, bei der das Schaltelement ein TFT (32) mit Inverted Staggered-Aufbau ist.

5. Vorrichtung nach Anspruch 3 oder 4, umfassend eine erste Schutzschicht (36) zwischen einem Schaltelement und der ersten Planarisierungsschicht.

6. Vorrichtung nach Anspruch 5, umfassend eine zweite Schutzschicht (42) zwischen der zweiten Planarisierungsschicht und dem Scintillator.

7. Vorrichtung nach einem der Ansprüche 1 bis 6, bei der eine Kantenfläche (37b) der ersten Planarisierungsschicht und eine Kantenfläche (39b) der zweiten Planarisierungsschicht sich verjüngend ausgebildet sind.

8. Vorrichtung nach einem der Ansprüche 1 bis 7, umfassend eine Abdichtungsschicht (23) zum Abdecken der Oberfläche des Scintillators und der Kantenfläche der zweiten Planarisierungsschicht.

9. Vorrichtung nach Anspruch 8, umfassend eine Lichtreflexionsschicht (24) über derAbdichtungsschicht.

10. Vorrichtung nach einem der Ansprüche 1 bis 9, bei der die photoelektrische Wandlerplatte, der Scintillator und die Trägerplatte in einem Gehäuse (14) mit Monocoque-Struktur aufgenommen sind.

11. Vorrichtung nach einem der Ansprüche 1 bis 10, bei der das isolierende Substrat aus Glas gefertigt ist.

## Revendications

1. Dispositif de détection d'image radiographique (10), comprenant un panneau de conversion photoélectrique (21) et un scintillateur (20), le panneau de conversion photoélectrique et le scintillateur étant disposés dans cet ordre à partir d'un côté d'incidence de rayonnement, où un rayonnement provenant d'une source de rayonnement (70) est incident lors de d'imagerie,
(A) le panneau de conversion photoélectrique comprenant :
un substrat isolant (30) ;
une pluralité d'éléments de commutation (32) formés par-dessus le substrat isolant ;
un premier film de planarisation (37) formé de manière à recouvrir les éléments de commutation et présentant une surface planarisée ;
une pluralité de photodiodes (33) formées par-dessus le premier film de planarisation, et
un second film de planarisation (39) formé de manière à recouvrir les photodiodes et le premier film de planarisation et présentant une surface planarisée ;
(B) le scintillateur comprenant :
une couche de cristaux sans colonnes (20b) et une pluralité de cristaux en colonnes (20a) formée sur la couche de cristaux sans colonnes,
la couche de cristaux sans colonnes étant disposée sur le côté de panneau de conversion photoélectrique par rapport aux cristaux en colonnes,
de l'iodure de césium déposé par évaporation sous vide sur une zone de dépôt par évaporation sous vide (50), la zone de dépôt par évaporation sous vide s'étendant par-dessus le second film de planarisation et étant située dans un premier bord (37a) du premier film de planarisation et un second bord (39a) du second film de planarisation et couvrant une zone (51) où sont formés les éléments de commutation et les photodiodes, et
(C) une plaque support (12) pour supporter le scintillateur, la plaque support étant fixée sur une surface du scintillateur où le panneau de conversion photoélectrique n'est pas disposé,
**caractérisé en ce que** le panneau de conversion photoélectrique présente une ligne de polarisation (90) pour fournir une tension de polarisation à chaque dite photodiode, et la ligne de polarisation est formée entre chaque dite photodiode et le substrat isolant.

2. Dispositif de détection d'image radiographique selon la revendication 1, dans lequel le premier bord du premier film de planarisation se trouve dans le second bord du second film de planarisation.

3. Dispositif de détection d'image radiographique selon la revendication 1 ou 2, dans lequel une pluralité de pixels (31) contenant chacun le un élément de commutation et la une photodiode sont agencés en une matrice par-dessus le substrat isolant.

4. Dispositif de détection d'image radiographique selon la revendication 3, dans lequel l'élément de commutation est un transistor à couches minces (Thin Film Transistor, TFT, 32) de type à décalage inversé.

5. Dispositif de détection d'image radiographique selon la revendication 3 ou 4, comprenant un premier film de protection (36) entre l'élément de commutation et le premier film de planarisation.

6. Dispositif de détection d'image radiographique selon la revendication 5, comprenant un second film de protection (42) entre le second film de planarisation et le scintillateur.

7. Dispositif de détection d'image radiographique selon l'une quelconque des revendications 1 à 6, dans lequel une face de bord (37b) du premier film de planarisation et une face de bord (39b) du second film de planarisation présentent un amincissement progressif.

8. Dispositif de détection d'image radiographique selon l'une quelconque des revendications 1 à 7, comprenant un film d'étanchéité (23) pour recouvrir la surface du scintillateur et la face de bord du second film de planarisation.

9. Dispositif de détection d'image radiographique selon la revendication 8, comprenant un film de réflexion de la lumière (24) par-dessus le film d'étanchéité.

10. Dispositif de détection d'image radiographique selon l'une quelconque des revendications 1 à 9, dans lequel le panneau de conversion photoélectrique, le scintillateur, et la plaque support sont logés dans un logement (14) avec une structure monocoque.

11. Dispositif de détection d'image radiographique selon l'une quelconque des revendications 1 à 10, dans lequel le substrat isolant est fabriqué en verre.
